# DEMANDE DE BREVET EUROPEEN

(11) **EP 4 112 017 A1**
(43) Date de publication de la demande: **04.01.2023**
(21) Numéro de dépôt: 22177632.1
(22) Date de dépôt: 07.06.2022
(51) Int. Cl.: A61F 7/00, A61F 7/02

(54) **PLASTRON CHAUFFANT PERMETTANT DE LUTTER CONTRE L'HYPOTHERMIE ACCIDENTELLE**

(30) Priorité: 02.07.2021 FR 2107224
(71) Demandeur: Thermo-Trauma, 05100 Briancon (FR)
(72) Inventeur: SPADONI, Thomas, 05100 PUY SAINT ANDRE (FR); BERGON, Rémy, 07700 GRAS (FR)
(74) Mandataire: Med'inVent Consulting

(57) **Abrégé**

[L'invention concerne un plastron chauffant (100) destiné à être placé sur un corps d'un blessé. Un tel plastron chauffant (100) comporte une encolure (110) et une partie inférieure (120) épousant ledit corps du blessé respectivement au niveau du cou et du tronc dudit blessé. En outre, ledit plastron (100) comporte un élément chauffant (130) destiné à réchauffer ledit corps du blessé. Ledit élément chauffant (130) est positionné dans l'encolure (110) et au niveau de la partie inférieure (120).

## Description

### Domaine technique

La présente invention se rapporte à un plastron chauffant permettant de lutter contre l'hypothermie accidentelle des blessés, notamment en phase pré-hospitalière.

### Arrière-Plan Technologique

L'hypothermie accidentelle est une situation clinique fréquente et grave chez les blessés polytraumatisés et peut atteindre 30 à 50% des patients les plus graves. Cela est notamment décrit dans l'étude de Søreide K., intitulée Clinical and translational aspects of hypothermia in major trauma patients : from pathophysiology to prevention, prognosis and potential préservation et publiée dans la revue scientifique Injury de l'année 2014.

Par ailleurs, les conséquences d'une telle hypothermie sur les troubles de la coagulation sont responsables de près de 50 pourcents des décès dans les 48 heures suivant l'admission en réanimation, tel que détaillé dans les études :
- de Brown DJ, Brugger H, Boyd J, Paal P, intitulée Accidentai hypothermia et publiée dans la revue scientifique N Engl J Med en Novembre 2012 ;
- et/ou de Silfvast T, Pettilä V, intitulée Outcome from severe accidentai hypothermia in Southern Finland--a 10-year review et publiée dans la revue scientifique Resuscitation en Décembre 2003.

La prise en charge de cette hypothermie accidentelle constitue donc un enjeu majeur de la médecine d'urgence.

A ce jour, les systèmes disponibles sur le marché permettant cette prise en charge consistent en des systèmes passifs, isolants, évitant plus ou moins la déperdition de chaleur, tels que les couvertures de survie (couverture de survie de type Comed) ou en des systèmes de réchauffement actif.

Ces derniers peuvent correspondre à des couvertures utilisant l'énergie d'une réaction chimique telles que des chaufferettes libérant de la chaleur lorsqu'elles sont percutées, par exemple connues sous l'appellation commerciale « Ready-Heat II Torso Blanket ». De tels systèmes sont plus efficaces que les systèmes de réchauffement passif mais sont encombrants et à usage unique. Il existe d'autres systèmes de réchauffement actif utilisant notamment l'énergie électrique, par exemple les systèmes connus sous l'appellation commerciale « UniqueResc » de la société « Geratherm ». De tels systèmes fonctionnent sur secteur ou batterie externe, compliquant leur utilisation sur le terrain.

De plus, de tels systèmes de réchauffement actif présentent une taille et un encombrement pouvant rendre leur transport contraignant. En outre, ces systèmes ne sont pas adaptés d'un point de vue ergonomique pour venir au plus près des blessés. Leur positionnement gêne souvent les intervenants des premiers secours. En effet, de tels systèmes de réchauffement actif sont pour la plupart d'entre eux positionnés à l'extérieur des vêtements du blessé, rendant difficile la diffusion de la chaleur au plus près du corps à travers ces derniers.

### Résumé de l'invention

La présente invention vise à remédier aux inconvénients précités, notamment à proposer un plastron chauffant intégrant un système de chauffe, se fixant au niveau du cou du blessé par une encolure et épousant parfaitement le cou et le tronc du blessé (le thorax, l'abdomen et les flancs).

En effet, afin de prévenir ou de traiter l'hypothermie accidentelle, l'invention vise à réchauffer les blessés au niveau du tronc, là où le sang s'accumule pour protéger les organes nobles ainsi que les zones où les vaisseaux sont superficiels comme par exemple dans la région carotidienne, autrement dit au niveau du cou, permettant un réchauffement du sang irriguant le cerveau. Outre les effets sur la température du corps d'un blessé, la mise en place d'une source de chaleur sur le tronc d'un blessé a un effet rassurant, améliore le confort du blessé et diminue son stress, facilitant l'intervention des secouristes.

Ainsi, l'invention propose un plastron chauffant destiné à être placé sur un corps d'un blessé. Un tel plastron chauffant comporte une encolure et une partie inférieure épousant ledit corps du blessé respectivement au niveau du cou et du tronc dudit blessé. En outre, ledit plastron comporte un élément chauffant destiné à réchauffer ledit corps du blessé. Ledit élément chauffant est positionné dans l'encolure et au niveau de la partie inférieure.

L'élément chauffant peut consister en au moins une résistance chauffante souple formée de conducteurs électriques alimentés par des batteries et transformant l'énergie électrique en énergie thermique.

Le plastron chauffant peut comporter un textile multicouche se composant de deux couches externes en polyamide enduit d'un polymère polyuréthane et enserrant une couche chauffante. L'une des deux couches externes correspond à une face antérieure dudit plastron qui est en contact avec un milieu extérieur. L'autre des deux couches externes correspond à une face postérieure dudit plastron qui vient en contact avec le corps du blessé.

Une telle couche chauffante peut être constituée d'un circuit imprimé. Ledit circuit imprimé peut comprendre un substrat en polyester souple recouvert d'une couche conductrice souple en aluminium. À ce titre, la au moins une résistance chauffante est gravée sur ladite couche d'aluminium.

En complément, le circuit imprimé peut être recouvert d'une couche de protection en polyester.

Dans un mode de réalisation selon lequel ledit plastron chauffant comporte un textile multicouche, ce dernier peut comporter une couche d'isolation thermique comprise entre la couche externe de la face antérieure et la couche chauffante. À ce titre, ladite couche d'isolation thermique peut être constituée d'un laminé de cellulose et d'aluminium.

Dans un mode de réalisation selon lequel ledit plastron chauffant comporte des batteries, ledit plastron peut comporter en outre des poches au niveau du tiers inférieur dudit plastron. De telles poches peuvent être réparties symétriquement de part et d'autre d'une ligne médiane dudit plastron et sont destinées à recevoir les batteries.

Préférentiellement, ladite encolure est de forme rectangulaire et présente une largeur de sept centimètres et une longueur de trente-huit centimètres. Ladite encolure est agencée pour épouser le cou du blessé.

En complément, ladite encolure peut comporter un système d'attache rajoutant une longueur supplémentaire à ladite encolure et permettant d'attacher ledit plastron au cou du blessé.

Enfin, ledit plastron peut comporter une poche de rangement avec un système de fermeture, un sac de rangement dudit plastron et des câbles d'alimentation des batteries. Ladite poche de rangement est préférentiellement positionnée sur la partie inférieure dudit plastron et est destinée à contenir ledit sac de rangement et lesdits câbles d'alimentation.

### Brève description des dessins

L'invention sera mieux comprise et d'autres caractéristiques et avantages de celle-ci apparaîtront à la lecture de la description suivante de modes de réalisation particuliers de l'invention donnés à titre d'exemples illustratifs et non limitatifs et faisant référence aux dessins annexés, parmi lesquels :
La figure 1 montre une vue de la face postérieure d'un plastron chauffant selon l'invention,
La figure 2 montre le plastron chauffant selon l'invention en utilisation sur le corps d'un blessé,
La figure 3 montre une vue de la face antérieure du plastron chauffant selon l'invention,
La figure 4 montre une vue schématique selon une coupe AA de la figure 1,
La figure 5 montre une vue schématique en coupe du plastron chauffant selon un mode de réalisation de l'invention,
La figure 6 montre un mode de réalisation d'un élément chauffant du plastron chauffant selon l'invention.

### Description détaillée

Afin de simplifier la description, une même référence est utilisée dans différentes figures pour désigner un même objet. Ainsi, lorsque la description cite un objet référencé, cet objet pourra être identifié sur plusieurs figures. En outre, les figures ainsi que la description sont données à titre d'exemples non limitatifs de réalisation.

En préambule, dans la suite de la présente demande, le terme « face postérieure » correspond à la face d'un plastron chauffant venant en contact avec le corps d'un blessé et le terme « face antérieure » correspond à la face du plastron chauffant étant en contact avec un milieu extérieur lorsque le plastron chauffant est positionné sur un blessé.

L'invention consiste en un plastron chauffant 100 destiné à être placé sur un corps 200 d'un blessé, plus précisément sur le torse du blessé. Un tel dispositif 100 permet ainsi de traiter un blessé en hypothermie accidentelle et/ou de prévenir toute hypothermie d'un blessé en situation d'urgence et de premiers secours.

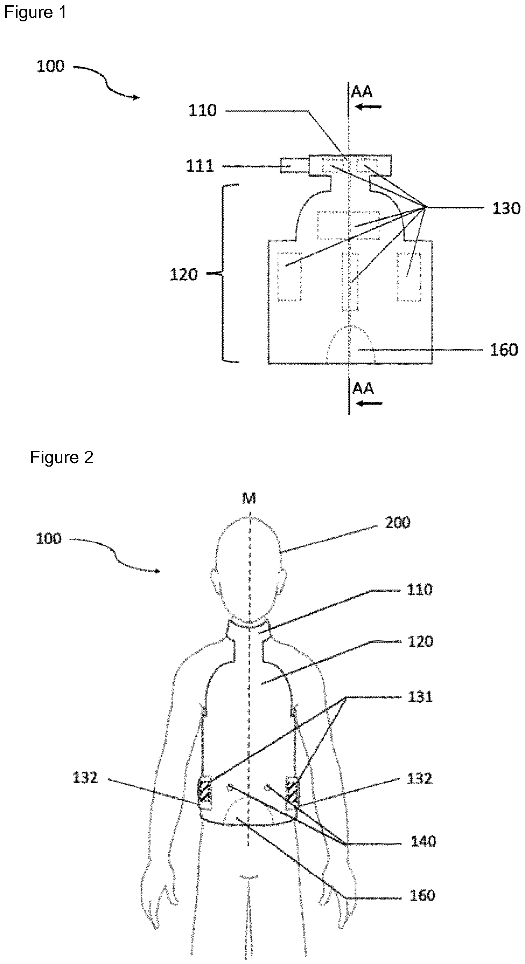

Un exemple de réalisation d'un plastron chauffant 100 conforme à l'invention est illustré de manière schématique en figure 1. A ce titre, la face dudit plastron chauffant 100 montrée sur la figure 1 correspond à la face postérieure.

Tel qu'illustré sur la figure 1, le plastron chauffant 100 conforme à l'invention comporte une encolure 110 destinée à épouser le cou du blessé et une partie inférieure 120 destinée à épouser le tronc du blessé. En outre, ladite encolure 110 et ladite partie inférieure 120 disposent toutes deux d'au moins un élément chauffant 130, réchauffant le corps 200 du blessé.

L'encolure 110 est une pièce en textile de forme rectangulaire agencée pour s'adapter au mieux au cou d'un blessé, quelle que soit la morphologie dudit blessé. À ce titre, une telle encolure 110 présente des dimensions comprises entre trois et quinze centimètres en largeur et entre trente et cinquante centimètres en longueur. Préférentiellement, ladite largeur est de sept centimètres et ladite longueur est de trente-huit centimètres pour correspondre à toutes les morphologies de patients.

En outre, une telle encolure 110 peut comporter un système d'attache 111 permettant de fixer le plastron chauffant 100 au cou du blessé tel qu'illustré en figures 1 et 2. En effet, un tel système 111 rajoute une longueur supplémentaire à ladite encolure 110 et permet ainsi d'entourer l'intégralité du cou dudit blessé. Le système d'attache 111 peut être réalisé de différentes façons, tel que par exemple, un système de fixation par fermeture auto-agrippante ou autrement nommé sous l'appellation anglo-saxonne « scratchs » de type connu sous la marque « Velcro ». Toutefois, l'homme du métier ne saurait limiter l'invention au système d'attache choisi. Tout autre type de système permettant d'être attaché rapidement au cou du blessé peut être envisagé, tel que par exemple un système de fermeture éclair ou de bouton pression, ou autres dispositifs similaires.

Ainsi, une fois attaché autour du cou du blessé, le plastron chauffant 100 peut être aisément déroulé sur le reste du corps 200 en tirant ladite partie inférieure 120 vers le bas, évitant ainsi les difficultés de positionnement et d'ajustement sur ledit corps 200. Ladite partie inférieure 120 vient alors se positionner naturellement sur le torse du blessé épousant parfaitement le thorax, l'abdomen et les flancs. Cela permet de positionner ledit plastron chauffant 100 sans mobiliser le blessé. Il en est de même pour le retrait dudit plastron 100 facilitant l'accès au thorax pour le personnel soignant, notamment dans les situations où un massage cardiaque doit être réalisé sur le blessé ou lorsqu'un dispositif de surveillance du rythme cardiaque avec électrode de type scope médical doit être posé sur le blessé.

La figure 1 présente ledit plastron chauffant 100 avec six éléments chauffants 130 qui peuvent par exemple consister en des résistances chauffantes souples 130 réparties sur les zones stratégiques du cou et du tronc (thorax, abdomen, flancs). Cependant, un seul élément chauffant 130, consistant par exemple comme dit précédemment en une résistance chauffante 130, peut suffire. La ou les résistance(s) chauffante(s) 130 est (sont) formée(s) de conducteurs électriques longs et fins présentant ainsi une résistance électrique importante. La circulation d'un courant dans lesdits conducteurs transforme l'énergie électrique en énergie thermique par effet joule.

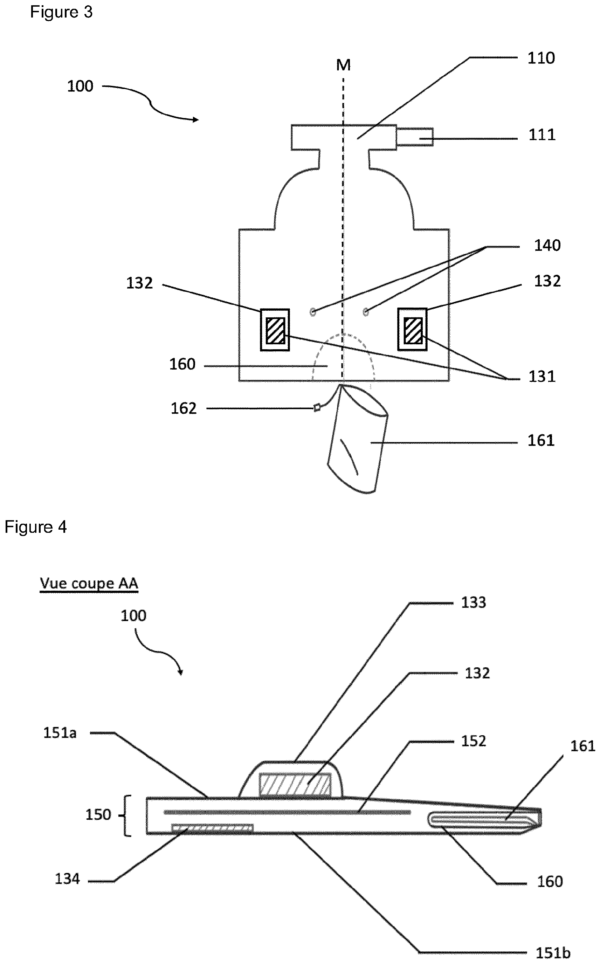

En outre, les conducteurs électriques sont alimentés par une (ou plusieurs) batterie(s) 131, telle(s) qu'illustrée(s) en figures 2 et 3. De telles batteries 131 sont préférentiellement des batteries en Lithium-Ion ou tout autre type de batteries capables d'assurer une telle alimentation électrique. L'autonomie des batteries 131 recherchée est d'au moins 4 heures afin de laisser aux secours le temps nécessaire pour rejoindre l'hôpital. Ainsi, un tel élément chauffant 130 alimenté par des batteries 131 intégrées au plastron 100 permet de proposer aux secouristes un dispositif autonome sans aucune autre connectique ni source d'alimentation externe nécessaire le temps de l'intervention d'urgence. En outre, il est préférable que de telles batteries 131 soient équipées d'un système de protection contre les courts circuits, la surchauffe et la haute tension afin d'éviter tout risque de brûlure du blessé.

Les batteries 131, telles qu'illustrées sur les figures 2 et 3 peuvent être positionnées dans des poches 132 pour batteries. Lesdites poches 132 sont situées au niveau de la partie inférieure 120 dudit plastron 100, de préférence au niveau du tiers inférieur dudit plastron 100 et réparties symétriquement de part et d'autre d'une ligne médiane M du corps 200 du blessé. De telles poches 132 peuvent disposer d'ouvertures rapides, par exemple de type auto-agrippante ou autrement nommé sous l'appellation anglo-saxonne « scratchs », facilitant l'accessibilité des batteries 131 en cas de nécessité de les remplacer par exemple. Le positionnement des batteries 131 permet, de par leur poids, de déplier entièrement par gravité le plastron chauffant 100 vers le bas, facilitant par la suite la mise en place et le maintien de la position dudit plastron 100 sur le corps 200 du blessé notamment au niveau des flancs du blessé.

Le ou les élément(s) chauffant(s) 130 peu(ven)t être activé(s) par l'intermédiaire de boutons marche/arrêt 140, tels qu'illustrés en figures 2 et 3. De tels boutons 140 sont préférentiellement étanches et fixés sur la partie inférieure 120 dudit plastron 100 afin d'être facilement accessibles pour la mise en route dudit élément chauffant 130 et de façon à ne pas gêner les gestes de premiers secours.

De façon optionnelle, l'élément chauffant 130 peut être équipé d'un dispositif permettant de vérifier son bon fonctionnement et d'un témoin de charge des batteries 131. Ce dispositif peut être par exemple, de type bargraphe, diode électro luminescente autrement connue sous l'acronyme LED ou autre. En outre, au démarrage de l'élément chauffant 130, ce dispositif peut s'allumer ou s'éteindre en cas de dysfonctionnement. Il est ainsi possible de vérifier le bon fonctionnement dudit plastron 100 avant son utilisation et le niveau de charge des batteries 131.

Selon un mode de réalisation illustré en figure 4, le plastron chauffant 100 peut être composé d'un textile multicouche 150. À ce titre, un tel textile multicouche 150 est formé d'une première couche externe 151a correspondant à la face antérieure dudit plastron 100 et d'une deuxième couche externe 151b correspondant à la face postérieure dudit plastron 100. Lesdites couches 151a et 151b peuvent être cousues entre elles afin de proposer un textile multicouche 150 le plus fin et compact possible. Afin d'avoir une meilleure solidité de l'ensemble textile, les coutures sont préférentiellement situées dans des zones où il n'y a pas de résistance par exemple sur les bords ou sur une partie centrale dépourvue de résistance. Ledit élément chauffant 130 peut ainsi être inséré comme une couche chauffante 133 dans l'épaisseur du plastron chauffant 100 entre les deux couches externes 151a et 151b, tel qu'illustré en figure 4.

De telles couches externe 151a et 151b sont préférentiellement étanches afin de constituer une barrière aux liquides et de ne pas laisser passer les sécrétions. En outre, il est possible de les lessiver et nettoyer avec des produits de décontamination. Par ailleurs, lesdites couches 151a et 151b doivent répondre aux normes d'hygiène et de santé médicale. Au vu des différentes propriétés attendues, il est privilégié des couches 151a et 151b en polyamide enduit d'un polymère polyuréthane anti-inflammable, de type M1 selon les normes de sécurité incendie. Toutefois, l'invention ne saurait se limiter au choix du (ou des) matériau(x) pour les couches externes 151a et 151b. Tout autre type de matériau peut être envisagé tant qu'il répond au besoin exprimé et décrit précédemment. En complément, il est même envisageable que l'on puisse appliquer des traitements particuliers sur de telles couches pour renforcer certaines de leurs propriétés tel que par exemple un traitement bactéricide. Enfin, de telles couches 151a et 151b peuvent être identifiées par un code couleur afin d'éviter les confusions d'utilisation entre la face antérieure et la face postérieure dudit plastron 100. Par exemple, on peut utiliser du rouge/orange sur la couche 151b correspondant à la face postérieure et du bleu/noir sur la couche 151a correspondant à la face antérieure.

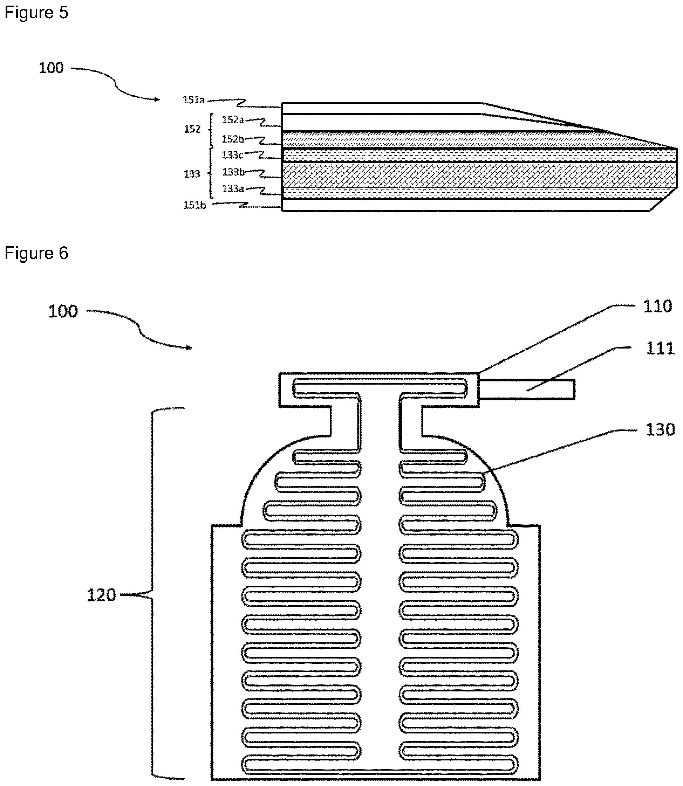

En complément, ledit textile multicouche 150 peut comporter une couche supplémentaire afin de concentrer la chaleur et de la renvoyer vers le corps 200 du blessé et ainsi de limiter les pertes thermiques. Cette couche autrement nommée couche d'isolation thermique 152 est comprise entre la couche externe de la face antérieure 151a et la couche chauffante 133, telle qu'illustrée en figure 4. Une telle couche d'isolation thermique 152 peut être constituée d'un laminé de cellulose 152a et d'aluminium 152b tel qu'illustré en figure 5.

Selon une première variante de réalisation de l'élément chauffant 130, illustrée en figure 5, pour laquelle le plastron chauffant 100 est composé du textile multicouche 150, l'élément chauffant 130 peut consister en la couche chauffante 133 insérée dans l'épaisseur dudit plastron 100, tel que décrit précédemment et illustré en figure 4. À ce titre, une telle couche chauffante 133 peut consister en un circuit électrique dont les conducteurs électriques sont réalisés sous la forme d'un circuit imprimé. Un tel circuit imprimé comprend au moins une couche isolante 133a recouverte d'une couche conductrice 133b sur laquelle la ou les résistance(s) chauffante(s) 130 est (sont) gravée(s). Préférentiellement, de telles couches 133a et 133b sont souples. Ladite couche isolante 133a peut être un substrat en polyester. Ladite couche conductrice 133b peut être en aluminium. Par ailleurs, afin d'assurer la protection dudit circuit imprimé, ce dernier peut être recouvert par une couche de protection 133c préférentiellement collée par exemple par laminage à chaud sur la couche conductrice 133b. Ladite couche de protection 133c est préférentiellement en polyester souple. En outre, une deuxième couche de protection 133c peut également être déposée, par exemple par collage, sur la couche isolante 133a.

Selon une autre variante de réalisation de l'élément chauffant 130, illustrée notamment en figure 6, l'élément chauffant 130 peut simplement consister en un ou plusieurs fil(s) électrique(s) souple(s) alimenté(s) par un courant provenant des batteries 131. Dans ce même exemple illustré en figure 6, un seul conducteur peut former une résistance chauffante 130 globale qui s'étend sur toute la surface du plastron 100, de l'encolure 110 à la partie inférieure 120. Une telle configuration permet d'homogénéiser la chauffe au sein dudit plastron 100 en optant pour une chauffe globale. A contrario, dans l'exemple décrit notamment en figure 1, la chauffe se concentre essentiellement sur les zones dudit plastron 100 où les six résistances chauffantes 131 sont positionnées.

En complément, l'élément chauffant 130 peut être relié à une carte électronique assurant la régulation de la température par la mesure en continu du courant et de la tension. En effet, la mesure du courant et de la tension permet de donner la mesure de la résistance qui est variable en fonction de la température. À ce titre, lorsque la résistance augmente la température augmente. Il est ainsi possible d'avoir la valeur de la température à partir de la valeur mesurée de la résistance. Cela permet d'éviter les surchauffes accidentelles et les risques de brûlures, de ralentir la chauffe lorsque la température ciblée au sein dudit plastron 100 est atteinte mais également de déclencher/d'accélérer la chauffe pour maintenir les batteries 131 à une température optimale de service dans des conditions extrêmes notamment en grand froid (évitant toute détérioration des batteries 131). Ceci permet ainsi d'économiser l'énergie nécessaire au niveau des batteries 131 et donc de rallonger la durée de fonctionnement de ces dernières. À ce titre, la puissance des batteries 131 peut être contrôlée par un système de régulation électronique relié à l'élément chauffant 130. Lesdites batteries 131 et l'élément chauffant 130 peuvent alors être reliés au système de régulation par des câbles solidarisés par des connectiques sécurisées et étanches afin d'éviter tout risque de débranchement et de court-circuit. En outre, il est possible que ledit plastron 100 dispose d'une interface telle qu'un écran permettant de donner des indications de consigne de température, d'afficher des messages et également le niveau des charges des batteries 131 ainsi que des boutons permettant de régler la consigne de chauffe. Une telle interface se situe préférentiellement sur la couche externe de la face antérieure dans une zone facilement accessible et visible pour l'utilisateur. Une telle interface peut également être reliée à un système d'alarme de type sonore permettant d'avertir en cas de dysfonctionnement de l'élément chauffant 130 et/ou des batteries 131 ou d'un niveau faible des batteries 131.

Par ailleurs, comme l'illustre la figure 3, ledit plastron chauffant 100 conforme à l'invention peut comporter une poche de rangement 160 disposant d'un système de fermeture rapide parmi ceux cités précédemment. Ladite poche de rangement 160 est située de préférence dans la partie inférieure 120 du plastron 100. Une telle poche de rangement 160 est destinée à contenir un sac de rangement 161 dudit plastron 100 ainsi que des câbles d'alimentation 162 des batteries 131. Toutefois, la présence de cette poche de rangement 160 est optionnelle. Ledit sac de rangement 161, préférentiellement étanche, pourra être fourni comme un élément à part.

Ledit sac de rangement 161 permet de ranger et de protéger ledit plastron 100 lorsque celui-ci n'est pas utilisé. Ainsi, il est possible pour les secouristes de transporter à pied ledit plastron chauffant 100 conservé dans le sac de rangement 161 ou de le porter sur un baudrier pour les interventions en milieu périlleux. Afin de ne pas être égaré, ledit sac de rangement 161 peut être par exemple cousu ou relié par un cordon audit plastron 100. Un tel sac de rangement 161 est préférentiellement constitué d'un textile étanche afin de protéger ledit plastron 100 contre les intempéries, notamment pour faire face à des conditions humides. En outre, ledit sac 161 se ferme préférentiellement par un système de serrage rapide de type cordon ou autre.

Les câbles d'alimentation 162 permettent de recharger les batteries 131, de type par exemple prise jack. Le positionnement desdits câbles 162 doit permettre de recharger les batteries 131 même lorsque le plastron 100 est replié et conservé dans son sac de rangement 161. La recharge des batteries 131 se fait préférentiellement par l'intermédiaire d'un chargeur externe, qui pourra disposer d'un système permettant d'évaluer le niveau de charge des batteries 131.

Il sera apprécié de l'homme du métier que la présente divulgation n'est pas limitée à ce qui est particulièrement montré et décrit ci-dessus. D'autres modifications peuvent être envisagées sans sortir du cadre de la présente invention définie par les revendications ci-annexées. ]

## Revendications

1. Plastron chauffant (100) destiné à être placé sur un corps (200) d'un blessé comportant une encolure (110) et une partie inférieure (120) épousant ledit corps (200) du blessé respectivement au niveau du cou et du tronc dudit blessé et un élément chauffant (130) destiné à réchauffer ledit corps (200) du blessé, **caractérisé en ce que** ledit élément chauffant (130) est positionné dans l'encolure (110) et au niveau de la partie inférieure (120).

2. Plastron chauffant (100) selon la revendication précédente, pour lequel l'élément chauffant (130) consiste en au moins une résistance chauffante souple (130) formée de conducteurs électriques alimentés par des batteries (131) et transformant l'énergie électrique en énergie thermique.

3. Plastron chauffant (100) selon l'une des revendications précédentes, comportant un textile multicouche (150) se composant de deux couches externes (151a, 151b) en polyamide enduit d'un polymère polyuréthane et enserrant une couche chauffante (133), une des deux couches externes (151a) correspondant à une face antérieure dudit plastron (100) étant en contact avec un milieu extérieur et l'autre des deux couches externes (151b) correspondant à une face postérieure dudit plastron (100) venant en contact avec le corps (200) du blessé.

4. Plastron chauffant (100) selon la revendication précédente, pour lequel la couche chauffante (133) est constituée d'un circuit imprimé comprenant un substrat en polyester (133a) souple recouvert d'une couche conductrice (133b) souple en aluminium, la au moins une résistance chauffante (130) étant gravée sur ladite couche d'aluminium (133b).

5. Plastron chauffant (100) selon la revendication précédente, pour lequel le circuit imprimé est recouvert d'une couche de protection (133c) en polyester.

6. Plastron chauffant (100) selon l'une des revendications 3 à 5, pour lequel ledit textile multicouche (150) comporte une couche d'isolation thermique (152) comprise entre la couche externe de la face antérieure (151a) et la couche chauffante (133), ladite couche d'isolation thermique (152) étant constituée d'un laminé de cellulose (152a) et d'aluminium (152b).

7. Plastron chauffant (100) selon l'une des revendications 2 à 6, comportant des poches (132) au niveau du tiers inférieur dudit plastron (100) et réparties symétriquement de part et d'autre d'une ligne médiane (M) dudit plastron (100), lesdites poches (132) étant destinées à recevoir les batteries (131).

8. Plastron chauffant (100) selon l'une des revendications précédentes, pour lequel ladite encolure (110) est de forme rectangulaire et présente une largeur de sept centimètres et une longueur de trente-huit centimètres, ladite encolure (110) étant agencée pour épouser le cou du blessé.

9. Plastron chauffant (100) selon l'une des revendications précédentes, pour lequel ladite encolure (110) comporte un système d'attache (111) rajoutant une longueur supplémentaire à ladite encolure (110) et permettant d'attacher ledit plastron (100) au cou du blessé.

10. Plastron chauffant (100) selon l'une des revendications 2 à 9, comportant une poche de rangement (160) avec un système de fermeture, un sac de rangement (161) dudit plastron (100) et des câbles d'alimentation (162) des batteries (131), ladite poche de rangement (160) étant positionnée sur la partie inférieure (120) dudit plastron (100) et destinée à contenir ledit sac de rangement (161) et lesdits câbles d'alimentation (162).
